(19) Europäisches Patentamt
European Patent Office
Office européen des brevets

(11) Numéro de publication : **0 117 780**
**B1**

(12) **FASCICULE DE BREVET EUROPÉEN**

(45) Date de publication du fascicule du brevet : **05.03.86**

(21) Numéro de dépôt : **84400158.6**

(22) Date de dépôt : **25.01.84**

(51) Int. Cl.[4] : **C 07 C148/00**, C 07 C149/30, C 07 C149/36, C 07 C149/34, C 07 C149/42

# (54) Procédé de préparation de perhalogénoalkylthioéthers.

(30) Priorité : **02.02.83 FR 8301593**

(43) Date de publication de la demande : **05.09.84 Bulletin 84/36**

(45) Mention de la délivrance du brevet : **05.03.86 Bulletin 86/10**

(84) Etats contractants désignés : **BE CH DE FR GB IT LI NL SE**

(56) Documents cités :
**EP-A- 0 052 559**
**FR-A- 1 350 036**
**Le dossier contient des informations techniques présentées postérieurement au dépôt de la demande et ne figurant pas dans le présent fascicule.**

(73) Titulaire : **RHONE-POULENC SPECIALITES CHIMIQUES**
**"Les Miroirs" 18, Avenue d'Alsace**
**F-92400 Courbevoie (FR)**

(72) Inventeur : **Tordeux, Marc**
**23, rue des Ormeaux**
**F-92260 Fontenay-aux-Roses (FR)**
Inventeur : **Wakselman, Claude**
**18, rue du Clos d'Alençon**
**F-91120 Villebon-sur-Yvette (FR)**

(74) Mandataire : **Cazes, Jean-Marie et al**
**RHONE-POULENC RECHERCHES Service Brevets Chimie et Polymères 25, quai Paul Doumer**
**F-92408 Courbevoie Cedex (FR)**

**EP 0 117 780 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention a pour objet un procédé de préparation de perhalogénoalkylthioéthers ; elle concerne plus particulièrement la préparation de perhalogénoalkylthioéthers par réaction d'un thiophénate avec un perhalogénoalcane.

On connaît dans l'art antérieur divers procédés permettant l'obtention de perhalogénoalkylthioéthers. On connaît en particulier J. Gen. Chem. USSR 1952, *22,* 2273 et 1954, *24,* 885 qui décrivent la préparation de divers trifluorométhylthiobenzènes par chloration du dérivé $—SCH_3$ correspondant en dérivé $—SCCl_3$ puis par fluoration de ce dernier en $—SCF_3$. Cette même méthode permet, par fluoration incomplète, d'obtenir les dérivés $—SCF_2Cl$ (voir Angew. Chem. Int. Ed, 1977, *16,* 735).

On peut encore citer J. Org. Chem. 1964, *29,* 895 qui décrit la condensation du chlorure de trifluorométhane sulfényle $CF_3SCl$ avec des organomagnésiens pour obtenir les dérivés $—SCF_3$.

Dans Synthésis 1975, 721 est décrit un procédé de préparation de dérivés $—SCF_3$, par réaction de $CF_3SCu$ avec l'iodure correspondant.

Un autre procédé de l'art antérieur consiste à faire réagir $CF_3I$ et un thiol sous irradiation ultraviolette dans de l'ammoniac liquide (J. Org. Chem. USSR 1977, *13,* 972).

On citera enfin la préparation de dérivés $SCF_2Br$ par action de $CF_2Br_2$ ou $CF_2BrCl$ sur des thiophénates dans des conditions de transfert de phase liquide-liquide, l'agent de transfert de phase étant un sel d'ammonium quaternaire (Tetrahedron Letters 1981, *22,* 323).

Ce dernier procédé ne permet pas la mise en œuvre de $CF_3Br$ et $CF_2Cl_2$ et donc ne permet pas la préparation des dérivés $—SCF_3$ et $SCF_2Cl$.

Les autres procédés mentionnés présentent des inconvénients qui sont peu compatibles avec une exploitation industrielle et qui sont, principalement, le nombre élevé d'étapes nécessaires, la mise en œuvre de produits chers et/ou toxiques comme $CF_3I$, $CF_3SCl$ et $CF_3SCu$, le passage par un organomagnésien dont l'homme de l'art connaît bien les inconvénients ou encore la mise en œuvre de réactions dans de l'ammoniac liquide.

La présente invention pallie ces inconvénients et propose un procédé de mise en œuvre simple permettant d'obtenir de bons rendements.

La présente invention a donc pour objet un procédé de préparation de perhalogénoalkylthioéthers caractérisé en ce que l'on fait réagir un thiophénate alcalin avec un composé de formule :

$$X_1CF_2X_2 \qquad (I)$$

dans laquelle $X_1$ représente le chlore ou le brome et $X_2$ représente le chlore, le fluor, le radical $CF_3$, le radical $CF_2Cl$ ou le radical $CFCl_2$, et quand $X_1$ représente le brome $X_2$ ne peut représenter le chlore et quand $X_1$ représente le chlore $X_2$ ne peut représenter ni le fluor ni le radical $CF_2Cl$, dans un solvant aprotique polaire non nitré sous une pression supérieure à 1 bar.

Selon un mode de mise en œuvre préférentiel de l'invention, le thiophénate alcalin répond à la formule :

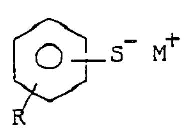

(II)

dans laquelle $M^+$ représente le sodium ou le potassium et R représente au moins un élément choisi parmi le groupe comprenant l'hydrogène et les radicaux alkyle ayant de 1 à 12 atomes de carbone, le radical phényle, les radicaux alkoxy ayant de 1 à 12 atomes de carbone, le radical phénoxy, les radicaux $NO_2$, CN, Cl, F, Br, I, $CF_3$, $CONR_1R_2$ et $NR_1R_2$ où $R_1$ et $R_2$, identiques ou différents, représentent chacun un hydrogène ou un radical alkyle ayant de 1 à 6 atomes de carbone.

On peut citer comme exemples de thiophénates alcalins les composés suivants : les para et ortho méthylthiophénates, les para et ortho méthoxythiophénates, les para et ortho halogénothiophénates, le méta et para trifluorométhylthiophénate, le méta aminothiophénate.

On préfère tout particulièrement utiliser les thiophénates de potassium.

L'invention est particulièrement bien adaptée à la mise en œuvre des composés de formule I suivants : $CF_3Br$, $CF_3—CF_2Br$, $CF_2Cl_2$ et $CF_2ClCFCl_2$.

On peut citer comme exemples de solvants aprotiques polaires non nitrés : l'acétonitrile, le diméthylformamide, le diméthylsulfoxyde, l'hexaméthylènephosphorotriamide, la N-méthylpyrrolidone, le diméthylacétamide, le sulfolane, les glymes.

On préfère mettre en œuvre : le diméthylformamide, le diméthylacétamide, la N-méthylpyrrolidone et le diméthylsulfoxyde.

La pression doit être supérieure à 1 bar. Elle sera généralement comprise entre 1,5 et 10 bars. L'homme de l'art sélectionnera dans cette fourchette la valeur la plus appropriée compte tenu à la fois des impératifs économiques du procédé et de la nature des réactifs mis en œuvre.

La température n'est pas un élément critique de l'invention. Elle peut varier dans de larges limites. On opérera de préférence à température ambiante, mais on peut aussi opérer à des températures pouvant aller jusqu'à 100 °C ou plus.

On utilise le thiophénate et le composé de formule I en quantités telles que le nombre de moles de composé de formule I par mole de thiophénate soit de préférence compris entre 1 et 10. Encore plus préférentiellement, il est compris entre 1 et 2.

La quantité de solvant mise en œuvre est au minimum la quantité nécessaire pour solubiliser le thiophénate. On utilise de préférence entre 0,5 et 1,5 litre de solvant par mole de thiophénate.

Les composés obtenus selon la présente invention sont des perhalogénoalkylthioéthers utiles, notamment, dans le domaine de la synthèse de composés ayant une activité pharmaceutique ou phytosanitaire. Ils y sont utilisés comme intermédiaires de synthèse.

Lorsque l'on met en œuvre un composé de formule II les produits obtenus selon le procédé de l'invention ont pour formule :

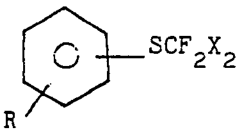

(III)

où R et $X_2$ ont la signification précédente.

L'invention va être maintenant plus complètement décrite à l'aide des exemples suivants. Ces derniers ne sauraient être considérés comme limitant le procédé de l'invention.

Exemple 1

Préparation du trifluorométhylthiobenzène

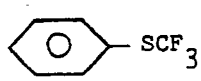 par action de $CF_3Br$ sur 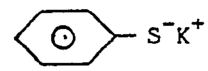

Dans un flacon en verre épais, on met 7,5 g (0,05 m) de thiophénate de potassium et 50 ml de diméthylformamide. Le flacon est placé dans un appareil de Parr. On fait le vide dans le flacon puis la bouteille est agitée sous une pression de 2,7 bars de bromotrifluorométhane. Au bout de 24 heures, il n'y a plus d'absorption de gaz. Le mélange est alors versé dans 100 ml d'acide chlorhydrique à 17 %. Une phase plus lourde que l'eau décante et est récupérée. La phase aqueuse est extraite à l'hexane auquel on incorpore la phase organique décantée. L'hexane est lavé avec de l'eau, séché sur carbonate de potassium, puis évaporé sous vide. Le résidu est distillé pour donner 5,5 g (0,031 m) de trifluorométhylthiobenzène (Eb 15000 Pa : 77-78 °C) ; Rdt : 62 % ; $Ø_F$ : −42 ppm.

Exemple 2

Préparation du méthyl-4 trifluorométhylthiobenzène.

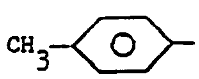 par action de $CF_3Br$ sur 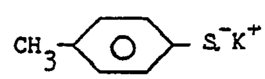

Dans un flacon en verre épais, on met 8,1 g (0,05 m) de méthyl-4 thiophénate de potassium et 50 ml de diméthylformamide. Le flacon est placé dans un appareil de Parr. On fait le vide dans le flacon ; puis la bouteille est agitée sous une pression de 2,7 bars de bromotrifluorométhane. Au bout de 24 heures, il n'y a plus d'absorption de gaz. Le mélange est alors versé dans 100 ml d'acide chlorhydrique à 17 %. Une phase plus lourde que l'eau décante et est récupérée. La phase aqueuse est extraite à l'hexane auquel on incorpore la phase organique décantée. L'hexane est lavé avec de l'eau, séché sur carbonate de potassium, puis évaporé sous vide. Le résidu est distillé pour donner 7,2 g (0,038 m) méthyl-4 trifluorométhylthiobenzène (Eb 2670 Pa : 70°) ; Rdt : 75 % ; $Ø_F$ : −43 ppm.

Exemple 3

Préparation du méthoxy-4 trifluorométhylthiobenzène.

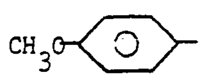 par action de $CF_3Br$ sur 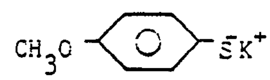

Dans un flacon en verre épais, on met 8,9 g (0,05 m) de méthoxy-4 thiophénate de potassium et 50 ml de diméthylformamide. Le flacon est placé dans un appareil de Parr. On fait le vide dans le flacon ; puis la

bouteille est agitée sous une pression de 2,7 bars de bromotrifluorométhane. Au bout de 24 heures, il n'y a plus d'absorption de gaz. Le mélange est alors versé dans 100 ml d'acide chlorhydrique à 17 %. Une phase plus lourde que l'eau décante et est récupérée. La phase aqueuse est extraite à l'hexane auquel on incorpore la phase organique décantée. L'hexane est lavé avec de l'eau, séché sur carbonate de potassium, puis évaporé sous vide. Le résidu est distillé pour donner 8,6 g (0,042 m) de méthoxy-4 trifluorométhylthiobenzène (Eb 2530 Pa : 90°) ; Rdt ; 83 % ; $\varnothing_F$ : −44 ppm.

Exemple 4

Préparation du chloro-4-trifluorométhylthiobenzène.

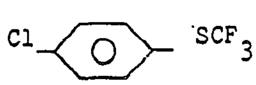 par action de $CF_3Br$ sur 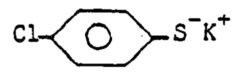

Dans un flacon en verre épais, on met 9,1 g (0,05 m) de chloro-4 thiophénate de potassium et 50 ml de diméthylformamide. Le flacon est placé dans un appareil de Parr. On fait le vide dans le flacon ; puis la bouteille est agitée sous une pression de 2,7 bars de bromotrifluorométhane. Au bout de 24 heures, il n'y a plus d'absorption de gaz. Le mélange est alors versé dans 100 ml d'acide chlorhydrique à 17 %. Une phase plus lourde que l'eau décante et est récupérée. La phase aqueuse est extraite à l'hexane auquel on incorpore la phase organique décantée. L'hexane est lavé avec de l'eau, séché sur carbonate de potassium, puis évaporé sous vide. Le résidu est distillé pour donner 3,6 g (0,017 m) de chloro-4 trifluorométhylthiobenzène (Eb 2530 Pa : 74°) ; Rdt : 34 % ; $\varnothing_F$ : −42 ppm.

Exemple 5

Préparation du chlorodifluorométhylthiobenzène.

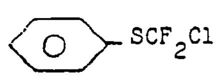 par action de $CF_2Cl_2$ sur 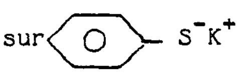

Dans un flacon en verre épais, on met 7,5 g (0,05 m) de thiophénate de potassium et 50 ml de diméthylformamide. Le flacon est placé dans un appareil de Parr. On fait le vide dans le flacon ; puis la bouteille est agitée sous une pression de 2,7 bars de dichlorodifluorométhane. Au bout de 24 heures, il n'y a plus d'absorption de gaz. Le mélange est alors versé dans 100 ml d'acide chlorhydrique à 17 %. Une phase plus lourde que l'eau décante et est récupérée. La phase aqueuse est extraite à l'hexane auquel on incorpore la phase organique décantée. L'hexane est lavé avec de l'eau, séché sur carbonate de potassium, puis évaporé sous vide. Le résidu est distillé pour donner 6 g (0,031 m) de chlorodifluorométhylthiobenzène (Eb 3330 Pa : 75°) ; Rdt : 62 % ; $\varnothing_F$ : −27 ppm.

Exemple 6

Préparation du méthyl-4 chlorodifluorométhylthiobenzène

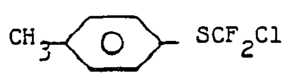 par action de $CF_2Cl_2$ sur 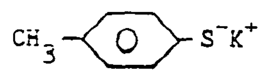

Dans un flacon en verre épais, on met 8,1 g (0,05 m) de méthyl-4 thiophénate de potassium et 50 ml de diméthylformamide. Le flacon est placé dans un appareil de Parr. On fait le vide dans le flacon ; puis la bouteille est agitée sous une pression de 2,7 bars de dichlorodifluorométhane. Au bout de 24 heures, il n'y a plus d'absorption de gaz. Le mélange est alors versé dans 100 ml d'acide chlorhydrique à 17 %. Une phase plus lourde que l'eau décante et est récupérée. La phase aqueuse est extraite à l'hexane auquel on incorpore la phase organique décantée. L'hexane est lavé avec de l'eau, séché sur carbonate de potassium, puis évaporé sous vide. Le résidu est distillé pour donner 4,6 g (0,022 m) méthyl-4 chlorodifluorométhylthiobenzène (Eb 2400 Pa : 87°) ; Rdt : 44 % ; $\varnothing_F$ : −27 ppm.

Exemple 7

Préparation du méthyl-4 dichloro-2',2' trifluoro-1',1',2' éthylthiobenzène.

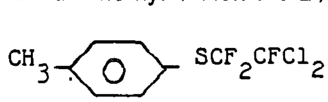 par action de $ClCF_2CFCl_2$ sur 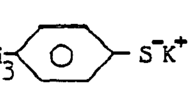

Dans un flacon en verre épais, on met 8,1 g (0,05 m) de méthyl-4 thiophénate de potassium, 50 ml de diméthylformamide et 9,4 g (0,05 m) de trichloro-1,1,2 trifluoro-1,2,2 éthane. Le flacon est fermé et agité sous pression autogène pendant 24 h. Le mélange est alors versé dans 100 ml d'HCl à 17 %. Une phase

plus lourde que l'eau décante et est récupérée. La phase aqueuse est extraite à l'hexane auquel on incorpore la phase organique décantée. L'hexane est lavé avec de l'eau, séché sur carbonate de potassium, puis évaporé sous vide. Le résidu est distillé pour donner 4,7 g (0,017 m) de méthyl-4 dichloro-2',2' trifluoro-1',1',2' éthylthiobenzène (Eb 53 Pa : 81°) ; Rdt : 34 % ; $Ø_F$ : −68 ppm (1F), −83 ppm (2F) ; $J_{FF}$ = 13,2 Hz.

Exemple 8

Préparation du méthoxy-2 trifluorométhylthiobenzène

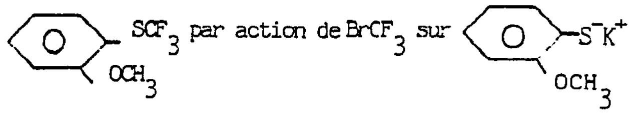


Dans un flacon en verre épais, on met 2,1 g (0,012 m) de méthoxy-2 thiophénate de potassium et 10 ml de diméthylformamide. Le flacon est placé dans un appareil de Parr. On fait le vide dans le flacon puis la bouteille est agitée sous une pression de 2,7 bars de bromotrifluorométhane. Au bout de 24 heures, il n'y a plus d'absorption de gaz. Le mélange est alors versé dans 20 ml d'acide chlorhydrique à 17 %. Une phase plus lourde que l'eau décante et est récupérée. La phase aqueuse est extraite à l'hexane auquel on incorpore la phase organique décantée. L'hexane est lavé avec de l'eau, séché sur carbonate de potassium, puis évaporé sous vide. Le résidu est distillé pour donner 1,0 g (0,048 m) de méthoxy-2 trifluorométhylthiobenzène (Eb 2670 Pa : 87 °C) ; Rdt : 40 % ; Ø : −42 ppm.

Exemple 9

Préparation du pentafluoroéthylthiobenzène

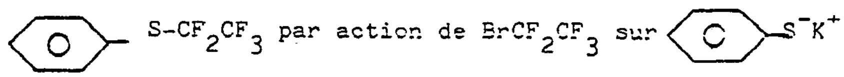


Dans un flacon en verre épais, on met 2,8 g (0,02 m) de thiophénate de potassium et 20 ml de diméthylformamide. Le flacon est placé dans un appareil de Parr. On fait le vide dans le flacon, puis la bouteille est agitée sous une pression de 1,5 bar de bromopentafluoroéthane. Au bout de 24 heures il n'y a plus d'absorption de gaz. Le mélange est alors versé dans 40 ml d'acide chlorhydrique à 17 %. Une phase plus lourde que l'eau décante et est récupérée. La phase aqueuse est extraite à l'hexane auquel on incorpore la phase organique décantée. L'hexane est lavé avec de l'eau, séché sur carbonate de potassium puis évaporé sous vide. Le résidu est distillé pour donner 1,5 g (0,0066 m) de pentafluoroéthylthiobenzène (Eb 3730 Pa : 53 °C) ; Rdt : 33 % ; $Ø_F$ −83 ppm (3F) ; −92 ppm (2F) ; $J_{FF}$ = 3, 0Hz.

Exemple 10

Préparation de l'acétamido-4 trifluorométhylthiobenzène.

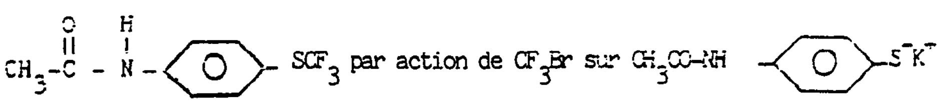


Dans un flacon en verre épais, on met 9,7 g (0,047 m) de acétamido-4 thiophénate de potassium et 50 ml de diméthylformamide. Le flacon est placé dans un appareil de Parr. On fait le vide dans le flacon puis la bouteille est agitée sous une pression de 2,7 bars de bromotrifluorométhane. Au bout de 24 heures, il n'y a plus d'absorption de gaz. Le mélange est alors versé dans 100 ml d'eau. Le solide est filtré, rincé plusieurs fois à l'eau, pour éliminer le DMF. Puis l'acétamido-4 trifluorométhylthiobenzène est dissous dans l'éther. L'éther est séché sur sulfate de magnésium, puis évaporé sous vide. On obtient ainsi 1 g (0,0043 m) d'acétamido-4 trifluorométhylthiobenzène ; F : 183° ; Rdt : 9 % ; $Ø_F$ : −44 ppm.

Exemple 11

Préparation de l'amino-3 trifluorométhylthiobenzène

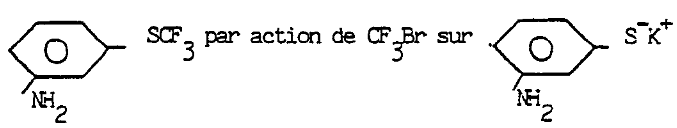

Dans un flacon en verre épais, on met 3,3 g (0,02 m) d'amino-3 thiophénate de potassium et 20 ml de diméthylformamide. Le flacon est placé dans un appareil de Parr. On fait le vide dans le flacon puis la bouteille est agitée sous une pression de 2,7 bars de bromotrifluorométhane. Au bout de 24 heures, il n'y a plus d'absorption de gaz. Le mélange est alors versé dans 40 ml d'eau. La phase aqueuse est extraite à l'hexane. L'hexane est séché sur carbonate de potassium, puis évaporé sous vide. Le résidu est distillé pour donner 0,9 g (0,0045 m) d'amino-3 trifluorométhylthiobenzène (Eb 4270 Pa : 112 °C) ; Rdt : 23 % ; $\varnothing_F$ : −42 ppm.

Exemple 12

Préparation du dichloro-2,2 trifluoro-1,1,2 éthylthio benzène.

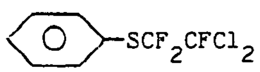 par action de $ClCF_2CFCl_2$ sur 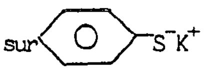

Dans un flacon en verre épais, on met 3,0 g (0,02 m) de thiophénate de potassium, 20 ml de diméthylformamide et 3,8 g (0,02 m) de trichloro-1,1,2 trifluoro-1,2,2 éthane. Le flacon est fermé et agité sous pression autogène pendant 24 h. Le mélange est alors versé dans 40 ml d'acide chlorhydrique à 17 %. Une phase plus lourde que l'eau décante et est récupérée. La phase aqueuse est extraite à l'hexane auquel on incorpore la phase organique décantée. L'hexane est lavé avec de l'eau, séché sur carbonate de potassium, puis évaporé sous vide. Le résidu est distillé pour donner 2,9 g (0,011 m) de dichloro-2,2 trifluoro-1,1,2 éthylthiobenzène (Eb 66 Pa : 56°) ; Rdt : 55 % ; $\varnothing_F$ : −68 ppm (1F), −82 ppm (2F) $J_{FF}$ = 13,4 Hz.

Exemple 13

Préparation du méthyl-4 dichloro-2,2 trifluoro-1,1,2 éthylthiobenzène.

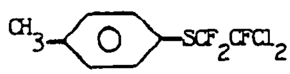 par action de $ClCF_2CFCl_2$ sur 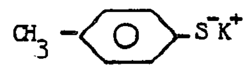

Dans un flacon en verre épais, on met 1,6 g (0,01 m) de méthyl-4 thiophénate de potassium, 10 ml de diméthylsulfoxyde et 1,9 g (0,01 m) de trichloro-1,1,2 trifluoro-1,2,2 éthane. Le flacon est fermé et agité sous pression autogène pendant 24 h. Le mélange est alors versé dans 20 ml d'acide chlorhydrique à 17 %. Une phase plus lourde que l'eau décante et est récupérée. La phase aqueuse est extraite à l'hexane auquel on incorpore la phase organique décantée. L'hexane est lavé avec de l'eau, séché sur carbonate de potassium, puis évaporé sous vide. Le résidu est distillé pour donner 1,2 g (0,0045 m) de méthyl-4 dichloro-2,2 trifluoro-1,1,2 éthylthiobenzène (Eb 53 Pa : 81°) ; Rdt : 45 % ; $\varnothing_F$ : −68 ppm (1F), −83 ppm (2F) ; $J_{FF}$ = 13,2 Hz.

Exemple 14

Préparation du méthoxy-4 dichloro-2,2 trifluoro-1,1,2 éthylthiobenzène.

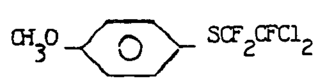 par action de $ClCF_2CFCl_2$ sur 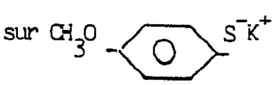

Dans un flacon en verre épais on met 1,8 g (0,01 m) de méthoxy-4 thiophénate de potassium, 10 ml de diméthylsulfoxyde et 1,9 g (0,01 m) de trichloro-1,1,2 trifluoro-1,2,2 éthane. Le flacon est fermé et agité sous pression autogène pendant 24 h. Le mélange est alors versé dans 20 ml d'acide chlorhydrique à 17 %. Une phase plus lourde que l'eau décante et est récupérée. La phase aqueuse est extraite à l'hexane auquel on incorpore la phase organique décantée. L'hexane est lavé avec de l'eau, séché sur carbonate de potassium, puis évaporé sous vide. Le résidu est distillé pour donner 1,3 g (0,0045 m) de méthoxy-4 dichloro-2,2 trifluoro-1,1,2 éthylthiobenzène (Eb 53 Pa : 87°) ; Rdt : 45 % ; $\varnothing_F$ : −68 ppm (1F), −84 ppm (2F) ; $J_{FF}$ = 13,2 Hz.

Exemple 15

Préparation du chloro-4 dichloro-2,2 trifluoro-1,1,2 éthylthiobenzène.

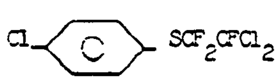 par action de $ClCF_2CFCl_2$ sur 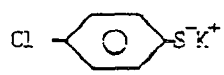

Dans un flacon en verre épais on met 1,8 g (0,01 m) de chloro-4 thiophénate de potassium, 10 ml de

diméthylsulfoxyde et 1,9 g (0,01) de trichloro-1,1,2 trifluoro-1,2,2 éthane. Le flacon est fermé et agité sous pression autogène pendant 24 h. Le mélange est alors versé dans 20 ml d'acide chlorhydrique à 17 %. Une phase plus lourde que l'eau décante et est récupérée. La phase aqueuse est extraite à l'hexane auquel on incorpore la phase organique décantée. L'hexane est lavé avec de l'eau, séché sur carbonate de potassium, puis évaporé sous vide. Le résidu est chromatographié sur silice avec le pentane comme éluant pour donner 0,4 g (0,0015 m) de chloro-4 dichloro-2,2 trifluoro-1,1,2 éthylthiobenzène (Eb 53 Pa 87°) ; Rdt : 15 % ; $Ø_F$ : −68 ppm (1F), −83 ppm (2F) ; $J_{FF}$ = 13,2 Hz.

Exemple 16

Préparation du chloro-4 trifluorométhylthiobenzène.

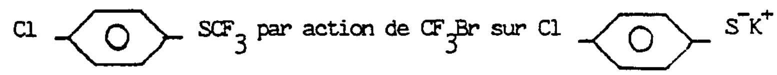


Dans un flacon en verre épais on met 3,6 g (0,02 m) de chloro-4 thiophénate de potassium et 20 ml de diméthylsulfoxyde. Le flacon est placé dans un appareil de Parr. On fait le vide dans le flacon puis la bouteille est agitée sous une pression de 2,7 bars de bromotrifluorométhane. Au bout de 24 heures, il n'y a plus d'absorption de gaz. Le mélange est alors versé dans 40 ml d'acide chlorhydrique à 17 %. Une phase plus lourde que l'eau décante et est récupérée. La phase aqueuse est extraite à l'hexane auquel on incorpore la phase organique décantée. L'hexane est lavé avec de l'eau, séché sur carbonate de potassium, puis évaporé sous vide. Le résidu est distillé pour donner 0,6 g (0,0030 m) de chloro-4 trifluorométhylthiobenzène (Eb 2530 Pa : 74 °C) ; Rdt : 15 % ØF : −42 ppm.

Exemple 17

Préparation du méthoxy-3 trifluorométhylthiobenzène.

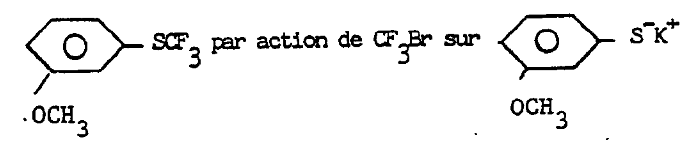


Dans un flacon en verre épais on met 3 g (0,017 m) de méthoxy-3 thiophénate de potassium et 20 ml de diméthylformamide. Le flacon est placé dans un appareil de Parr. On fait le vide dans le flacon puis la bouteille est agitée sous une pression de 2,7 bars de bromotrifluorométhane. Au bout de 24 heures, il n'y a plus d'absorption de gaz. Le mélange est alors versé dans 40 ml d'acide chlorhydrique à 17 %. Une phase plus lourde que l'eau décante et est récupérée. La phase aqueuse est extraite à l'hexane auquel on incorpore la phase organique décantée. L'hexane est lavé avec de l'eau, séché sur carbonate de potassium, puis évaporé sous vide. Le résidu est distillé pour donner 0,25 g (0,0012 m) de méthoxy-3 trifluorométhylthiobenzène (Eb 2670 Pa : 89°) ; Rdt : 7 % $Ø_F$ : −42 ppm.

Exemple 18

Préparation du trifluorométhyl-3 trifluorométhylthiobenzène.

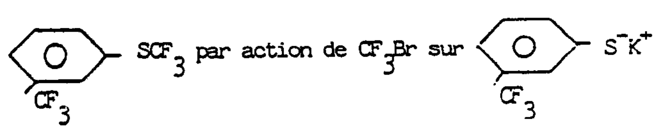


Dans un flacon en verre épais on met 4,3 g (0,02 m) de trifluorométhyl-3 thiophénate de potassium et 20 ml de diméthylformamide.

Le flacon est placé dans un appareil de Parr. On fait le vide dans le flacon puis la bouteille est agitée sous une pression de 2,7 bars de bromotrifluorométhane. Au bout de 24 heures, il n'y a plus d'absorption de gaz. Le mélange est alors versé dans 40 ml d'acide chlorhydrique à 17 %. Une phase plus lourde que l'eau décante et est récupérée. La phase aqueuse est extraite à l'hexane auquel on incorpore la phase organique décantée. L'hexane est lavé avec de l'eau, séché sur carbonate de potassium, puis évaporé sous vide. Le résidu est distillé pour donner 0,6 g (0,026 m) de trifluorométhyl-3 trifluorométhylthiobenzène (Eb 2270 Pa : 65°) ; Rdt : 13 % ; $Ø_F$ : −42 ppm ; −63 ppm.

**Revendications**

1. Procédé de préparation de perhalogénoalkylthioéthers caractérisé en ce que l'on fait réagir un

thiophénate alcalin avec un composé de formule :

$$X_1CF_2X_2 \quad (I)$$

dans laquelle $X_1$ représente le chlore ou le brome et $X_2$ représente le chlore, le fluor, le radical $CF_3$, le radical $CF_2Cl$ ou le radical $CFCl_2$ quand $X_1$ représente le brome $X_2$ ne peut représenter le chlore et quand $X_1$ représente le chlore, $X_2$ ne peut représenter ni le fluor ni le radical $CF_2Cl$, dans un solvant aprotique polaire non nitré sous une pression supérieure à 1 bar.

2. Procédé selon la revendication 1, caractérisé en ce que le thiophénate alcalin répond à la formule :

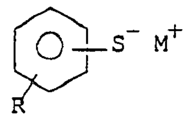

(II)

dans laquelle $M^+$ représente le sodium ou le potassium et R représente au moins un élément choisi parmi le groupe comprenant l'hydrogène et les radicaux alkyle ayant de 1 à 12 atomes de carbone, le radical phényle, les radicaux alkoxy ayant de 1 à 12 atomes de carbone, le radical phénoxy, les radicaux $NO_2$, CN, Cl, F, Br, I, $CF_3$, $CONR_1R_2$ et $NR_1R_2$ où $R_1$ et $R_2$, identiques ou différents, représentent chacun un hydrogène ou un radical alkyle ayant de 1 à 6 atomes de carbone.

3. Procédé selon l'une quelconque des revendications précédentes, caractérisé en ce que le thiophénate alcalin mis en œuvre est un thiophénate de potassium.

4. Procédé selon l'une quelconque des revendications précédentes, caractérisé en ce que le composé de formule I est $CF_3Br$, $CF_3CF_2Br$, $CF_2Cl_2$ ou $CF_2ClCFCl_2$.

5. Procédé selon l'une quelconque des revendications précédentes, caractérisé en ce que le solvant aprotique polaire non nitré est choisi parmi le groupe comprenant : le diméthylformamide, le diméthylacétamide, la N-méthylpyrrolidone et le diméthylsulfoxide.

6. Procédé selon l'une quelconque des revendications précédentes, caractérisé en ce que la pression est comprise entre 1,5 et 10 bars.

7. Procédé selon l'une quelconque des revendications précédentes, caractérisé en ce que la température est comprise entre la température ambiante et 100 °C.

8. Procédé selon l'une quelconque des revendications précédentes, caractérisé en ce que l'on met en œuvre entre 1 et 10 moles de composé de formule I par mole de thiophénate.

9. Procédé selon l'une quelconque des revendications précédentes, caractérisé en ce que l'on met en œuvre entre 0,5 et 1,5 litre de solvant par mole de thiophénate.

## Claims

1. Process for the preparation of perhalogenoalkylthioethers, characterised in that an alkali metal thiophenate is reacted with a compound of the formula

$$X_1CF_2X_2 \quad (I)$$

in which $X_1$ represents chlorine or bromine and $X_2$ represents chlorine, fluorine, the $CF_3$ radical, the $CF_2Cl$ radical or the $CFCl_2$ radical, but if $X_1$ represents bromine $X_2$ cannot represent chlorine and if $X_1$ represents chlorine $X_2$ cannot represent fluorine or the $CF_2Cl$ radical, in a non-nitrated polar aprotic solvent under a pressure greater than 1 bar.

2. Process according to Claim 1, characterised in that the alkali metal thiophenate corresponds to the formula

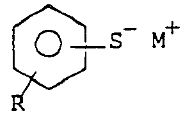

(II)

in which $M^+$ represents sodium or potassium and R represents at least one element chosen from among the group comprising hydrogen, alkyl radicals having from 1 to 12 carbon atoms, the phenyl radical, alkoxy radicals having from 1 to 12 carbon atoms, the phenoxy radical or the radicals $NO_2$, CN, Cl, F, Br, I, $CF_3$, $CONR_1R_2$ and $NR_1R_2$ where $R_1$ and $R_2$, which may be identical or different, each represent hydrogen or an alkyl radical having from 1 to 6 carbon atoms.

3. Process according to any one of the preceding claims, characterised in that the alkali metal thiophenate employed is a potassium thiophenate.

4. Process according to any one of the preceding claims, characterised in that the compound of the formula I is $CF_3Br$, $CF_3CF_2Br$, $CF_2Cl_2$ or $CF_2ClCFCl_2$.

5. Process according to any one of the preceding claims, characterised in that the non-nitrated polar

aprotic solvent is chosen from among the group comprising dimethylformamide, dimethylacetamide, N-methylpyrrolidone and dimethylsulfoxide.

6. Process according to any one of the preceding claims, characterised in that the pressure is between 1.5 and 10 bars.

7. Process according to any one of the preceding claims, characterised in that the temperature is between ambient temperature and 100 °C.

8. Process according to any one of the preceding claims, characterised in that between 1 and 10 moles of the compound of the formula I are employed per mole of thiophenate.

9. Process according to any one of the preceding claims, characterised in that between 0.5 and 1.5 litres of solvent are employed per mole of thiophenate.

**Patentansprüche**

1. Verfahren zur Herstellung von Perhalogenalkylthioethern, dadurch gekennzeichnet, daß man ein Alkalithiophenat mit einer Verbindung der Formel

$$X_1CF_2X_2 \quad \text{(I)}$$

in einem aprotischen polaren, nicht nitrierten Lösungsmittel unter einem Druck oberhalb von 1 bar umsetzt, in der $X_1$ Chlor oder Brom und $X_2$ Chlor, Fluor, das Radikal $CF_3$, das Radikal $CF_2Cl$ oder das Radikal $CFCl_2$ ist, wobei, wenn $X_1$ Brom bedeutet, $X_2$ nicht Chlor bedeuten und, wenn $X_1$ Chlor ist, $X_2$ weder Fluor noch das Radikal $CF_2Cl$ bedeuten kann.

2. Verfahren nach Anspruch 1, dadurch gekennzeichnet, daß das Alkalithiophenat die Formel besitzt

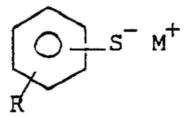

(II)

in der $M^+$ Natrium oder Kalium ist und R mindestens ein Element bedeutet, das aus der Gruppe ausgewählt ist, die Wasserstoff und die $C_1$-$C_{12}$-Alkylradikale, das Phenylradikal, die $C_1$-$C_{12}$-Alkoxyradikale, das Phenoxyradikal, die Radikale $NO_2$, CN, Cl, F, Br, I, $CF_3$, $CONR_1R_2$ und $NR_1R_2$ umfasst, wobei $R_1$ und $R_2$, die gleich oder verschieden sein können, jeweils ein Wasserstoffatom oder ein $C_1$-$C_6$-Alkylradikal bedeuten.

3. Verfahren nach Anspruch 1 oder 2, dadurch gekennzeichnet, daß das eingesetzte Alkalithiophenat ein Kaliumthiophenat ist.

4. Verfahren nach einem der Ansprüche 1 bis 3, dadurch gekennzeichnet, daß die Verbindung der Formel (I) $CF_3Br$, $CF_3CF_2Br$, $CF_2Cl_2$ oder $CF_2ClCFCl_2$ ist.

5. Verfahren nach einem der Ansprüche 1 bis 4, dadurch gekennzeichnet, daß das polare, nicht nitrierte aprotische Lösungsmittel ausgewählt ist aus der Gruppe, die umfasst : Dimethylformamid, Dimethylacetamid, N-Methylpyrrolidon und Dimethylsulfoxid.

6. Verfahren nach einem der Ansprüche 1 bis 5, dadurch gekennzeichnet, daß der Druck zwischen 1,5 und 10 bar liegt.

7. Verfahren nach einem der Ansprüche 1 bis 6, dadurch gekennzeichnet, daß die Temperatur zwischen Umgebungstemperatur und 100 °C liegt.

8. Verfahren nach einem der Ansprüche 1 bis 7, dadurch gekennzeichnet, daß man zwischen 1 und 10 Mol der Verbindung der Formel (I) pro Mol Thiophenat einsetzt.

9. Verfahren nach einem der Ansprüche 1 bis 8, dadurch gekennzeichnet, daß man zwischen 0,5 und 1,5 Liter Lösungsmittel pro Mol Thiophenat einsetzt.